# EUROPEAN PATENT SPECIFICATION

(11) **EP 2 492 351 B1**
(45) Date of publication and mention of the grant of the patent: **06.06.2018**
(21) Application number: 10749670.5
(22) Date of filing: 23.07.2010
(51) Int. Cl.: C12Q 1/00, G01N 27/30, G01N 33/487, G01N 33/543

(54) **ELECTROCHEMICAL SENSOR FOR THE DETECTION OF ANALYTES IN LIQUID MEDIA**
ELEKTROCHEMISCHER SENSOR FÜR DEN NACHWEIS VON ANALYTEN IN FLÜSSIGEN MEDIEN
DÉTECTEUR ÉLECTROCHIMIQUE POUR LA DÉTECTION D'ANALYTES DANS DES MILIEUX LIQUIDES

(30) Priority: 30.07.2009 ES 200930539; 23.12.2009 ES 200931247
(43) Date of publication of application: 29.08.2012
(73) Proprietor: Fundacion Cidetec, 20009 San Sebastian (ES)
(72) Inventor: OCHOTECO VAQUERO, Estibaliz, 20009 San Sebastian (ES); JUBETE DIEZ, Elena, 20009 San Sebastian (ES); POMPOSO ALONSO, José, Adolfo, 20009 San Sebastian (ES); GRANDE TELLERIA, Hans-Jurgen, 20009 San Sebastian (ES); LOAIZA, Oscar, A., 20009 San Sebastian (ES); CABAÑERO, German, 20009 San Sebastian (ES); ISTAMBOULIE, Georges, 66860 Perpignan (FR); NOGUER, Thierry, 66860 Perpignan (FR); MARTY, Jean, Louis, 66860 Perpignan (FR)
(74) Representative: Carpintero Lopez, Francisco
(86) International application number: PCT/ES2010/070509
(87) International publication number: WO 2011/012754

(56) References cited:
- WO-A1-2006/009324
- ISTAMBOULIE G ET AL: "The use of Artificial Neural Networks for the selective detection of two organophosphate insecticides: Chlorpyrifos and chlorfenvinfos", TALANTA, ELSEVIER, AMSTERDAM, NL LNKD- DOI:10.1016/J.TALANTA.2009.04.014, vol. 79, no. 2, 15 July 2009 (2009-07-15), pages 507-511, XP026221498, ISSN: 0039-9140 [retrieved on 2009-04-16]
- VALDES-RAMIREZ GABRIELA ET AL: "Acetylcholinesterase-based biosensors for quantification of carbofuran, carbaryl, methylparaoxon, and dichlorvos in 5% acetonitrile", ANALYTICAL AND BIOANALYTICAL CHEMISTRY, vol. 392, no. 4, October 2008 (2008-10), pages 699-707, XP19652856, ISSN: 1618-2642
- ISTAMBOULIE ET AL: "Highly sensitive detection of organophosphorus insecticides using magnetic microbeads and genetically engineered acetylcholinesterase", BIOSENSORS AND BIOELECTRONICS, ELSEVIER BV, NL LNKD- DOI:10.1016/J.BIOS.2007.06.022, vol. 23, no. 4, 17 October 2007 (2007-10-17), pages 506-512, XP022302121, ISSN: 0956-5663
- GERARD M ET AL: "Application of conducting polymers to biosensors", BIOSENSORS AND BIOELECTRONICS, ELSEVIER BV, NL LNKD- DOI:10.1016/S0956-5663(01)00312-8, vol. 17, no. 5, 1 May 2002 (2002-05-01), pages 345-359, XP002250769, ISSN: 0956-5663
- KROS A ET AL: "Poly(pyrrole) versus poly(3,4-ethylenedioxythiophene): implications for biosensor applications", SENSORS AND ACTUATORS B, ELSEVIER SEQUOIA S.A., LAUSANNE, CH LNKD- DOI:10.1016/J.SNB.2004.08.011, vol. 106, no. 1, 29 April 2005 (2005-04-29), pages 289-295, XP025328533, ISSN: 0925-4005 [retrieved on 2005-04-29]
- ISTAMBOULIE GEORGES ET AL: "Screen-printed poly(3,4-ethylenedioxythiophene) (PEDOT): A new electrochemical mediator for acetylcholinesterase-based biosensors", TALANTA, vol. 82, no. 3, 8 June 2010 (2010-06-08), pages 957-961, XP7915318, ISSN: 0039-9140

## Description

### Field of the Invention

The invention relates to the field of electrochemical sensors for the detection of analytes in liquid media. The invention particularly relates to electrochemical sensors including sensor electrodes based on low-cost conductive materials (carbonaceous materials and polythiophenes) which, deposited on a suitable support with an optimal geometry, allow the detection of minimum concentrations of compounds in liquid media by means of electrochemical techniques. Thus, the electrochemical sensor of the invention combines minimum limits of detection and, at the same time, low production costs.

### Background of the Invention

The environmental, food and agriculture and medical-healthcare field require efficient control and detection tools which, applied to specific problems, allow an improvement in the quality of life. Thus, it is increasingly necessary in the environmental field to control pesticides and toxic substances in water; and in the food field to control pesticides or pathogens in foods before their introduction in the market, as well as to rapidly detect the deterioration of foods. Likewise, in the medical-healthcare field the identification of the presence of foreign organisms in the human body and their concentration, as well as the early diagnosis of diseases, are essential.

Traditional analytical methods are based on liquid chromatography, gas chromatography or mass spectrometry. Generally, they are very reliable methods, with high degrees of detection. However, they are expensive and excessively slow methods. They are furthermore based on collecting samples for a subsequent analysis, so they do not allow an in-line control. It is therefore necessary to have analytical tools which require minimum sample preparation, which enable rapid measurement, which are portable and applicable *in situ,* and which allow acting in time against adverse situations.

These technologies must meet two requirements. Firstly, a high degree of sensitivity, this being a limiting factor and, secondly, that these degrees of sensitivity are obtained based on low-cost portable technologies which can be accessed by multiple users.

The European Union has established a maximum limit of pesticide residues in foods of about 0.1 micrograms per kilogram of food. Thus, the control and detection demands derived from this regulation are enormous. The development of new control and monitoring tools allowing not only the detection of these trace amounts of compound, but also a detection technology which can be used in any time and place, is demanded. In other words, it must be within the reach of farmers, food and agriculture companies, pesticide sellers, laboratories or any agent interested in controlling the presence of pesticides in fresh fruits and vegetables.

In relation to the medical-healthcare sector, technologies for detecting foreign organisms in the human body and their concentration for the early diagnosis of diseases are required. The higher the degree of sensitivity, the earlier the diagnosis will be available, thus allowing a suitable control and treatment. Low-cost technologies will in turn favor regular use in the doctor's office, or even a personal or domestic diagnosis, preventing the need for expensive and slow analyses in hospital laboratories.

Different electrochemical sensors based on the deposition of several tracks of material on an insulating substrate such as plastic or ceramic have been described in the state of the art. The tracks can be deposited by means of screen printing, lithography, vapor state deposition, spraying or similar deposition techniques. The first layer consists of a conductive metal material defining the geometry of the electrodes (working electrode or electrodes or sensors, reference electrode and counter electrode). This first layer of conductive metal material is usually made of silver, although other possible metal materials such as gold, platinum, palladium, copper or tungsten have been described (US 5,120,420, US 5,798,031, US 2005/0183953 A1, WO 2007/026152, US 2007/0080073). The second layer usually consists of a conductive material deposited on the working electrode and on the counter electrode which can be a metal material such as a paste based on gold, silver, platinum, palladium, copper or tungsten, for example; or a non-metal material such as a paste manufactured based on a carbon material (graphite or carbon black, for example) (US 5,120,420, US 5,798,031, US 2005/0183953 A1, WO 2007/026152, US 2007/0080073), or based on a conductive polymer (WO 2007/026152). In some cases, there is a third layer which usually consists of an Ag/AgCl paste, deposited only on the reference electrode (US 2005/0183953 A1). Likewise, on the working electrode there can be deposited a fourth layer of insulating material, typically of polymer material which can be a polymer gel (cellulose, poly(vinyl alcohol), gelatin, Tween-20, Triton X-100, Surfynol, etc.) for the physical entrapment of a biological compound participating in the electrochemical detection (US 2005/0183953 A1). This biological compound is responsible for the specificity and detection by means of electrochemical transduction and can be an enzyme, proteins, oligonucleotides, polynucleotides or vitamins, for example (US 2005/0183953 A1, US 5,120,420, US 5,798,031, FR 2 798 145).

This final layer of material in the working electrode can incorporate a mediator. The mediator acts by accepting electrons from the enzyme or donating electrons thereto once the electrochemical reaction has occurred. Thus, in some cases, the mediator can act by regenerating the oxidoreductase enzyme. The mediators described are transition metal complexes such as derivatives of ferrocene or ferrocyanides (US 5,653,863), but they can also be benzoquinones and naphthoquinones (US 4,746,607), nitrous compounds or hydroxylamines (EP 0 354 441), flavins, phenazines, phenothiazines, or indophenols (EP 0 330 517).

The use of polythiophenes as mediator agents in the manufacture of electrochemical sensors, a family of intrinsic conductive polymers with high stability and which can be processed from aqueous dispersions, has not been described in the state of the art. Patents US 4,959,430 and US 4,987,042 describe different processes for preparing dispersions based on poly(ethylenedioxythiophene) and patents US 5,766,515 and US 5,370,981 describe their use in the form of a transparent electrode in electroluminescent devices and for preparing antistatic plastics, respectively. Likewise, document FR 2 798 145 describes the use of polythiophenes, among many other electrochemically synthesized conductive polymers, as a support for anchoring specific recognition probes, since they contain functional groups in which said probes bind. On the other hand, document WO 2007/026152 describes the use of polythiophenes, among other conductive polymers, as a component of the electrode, substituting the carbon material in the second layer, but not as a mediator.

Likewise, electrochemical sensors which dispense with the first layer of conductive metal material, giving rise to a low-cost technology, have not been described either.

Therefore, there is still a need in the state of the art for alternative low-cost electrochemical sensors which do not incorporate expensive materials and the design of which enables the detection of analytes with maximum sensitivity.

Regardless of the nature of the materials forming the electrochemical sensor, the latter can include noble metal nanoparticles in its structure. Noble metal nanoparticles are the object of great interest in the field of chemistry, biology, medicine, etc., due to their thermal, electronic and optical properties. Thus, gold nanoparticles are of special interest given that they have a large surface area and suitable surface chemistry for the controlled immobilization of oligonucleotides (M. Paulose et al. Journal of Nanoscience and Nanotechnology 2003, 3, 341*;* S. Guo et al. Analitycal Chimica Acta 2007, 598, 181*;* M.Q.Wang et al. Chinese Journal of Analytical Chemistry 2008, 36, 890).

On the other hand, magnetic spheres are one of the latest tools used in the biodetection of DNA and proteins since they can be easily separated from a liquid phase with a magnet and be dispersed again when the magnet is removed. This fact enables the hybridization process to take place outside the surface of the electrode, thus preventing non-specific absorptions of the biomolecules in the detection surface of the sensor (O.A. Loaiza et al. Analytical Chemistry 2008, 80, 7*;* J. Wang et al. Talanta 2002, 56, 8).

The electrochemical sensor of the present invention uses polythiophenes as mediators and a first layer based on carbonaceous material, allowing the detection of analytes in a liquid medium with a high degree of sensitivity and with a lower cost and enabling the analysis of minimum concentrations of these analytes at any time, in any place and by multiple users. Furthermore, the authors of the present invention contemplate the possibility of the layer of polymer gel, located on the layer of polythiophene, being substituted with a layer of magnetic nanoparticles which are functionalized, i.e., incorporating a biological compound responsible for the detection covalently bonded in their surface.

### Object of the Invention

The object of the present invention is therefore to provide an electrochemical sensor for the detection of analytes in liquid media.

Another object of the present invention is a process for preparing said electrochemical sensor.

### Brief Description of the Drawings

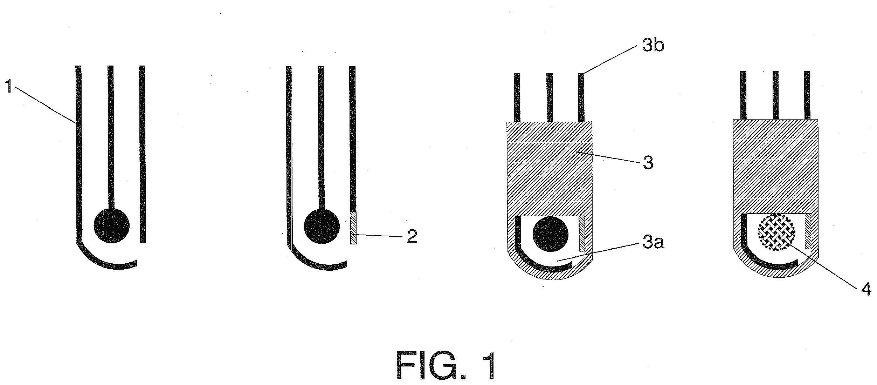

Figure 1 shows the top view of the electrochemical sensor of the invention incorporating a polythiophene as a mediator and a first layer based on carbonaceous material, in which its multilayer structure can be seen.

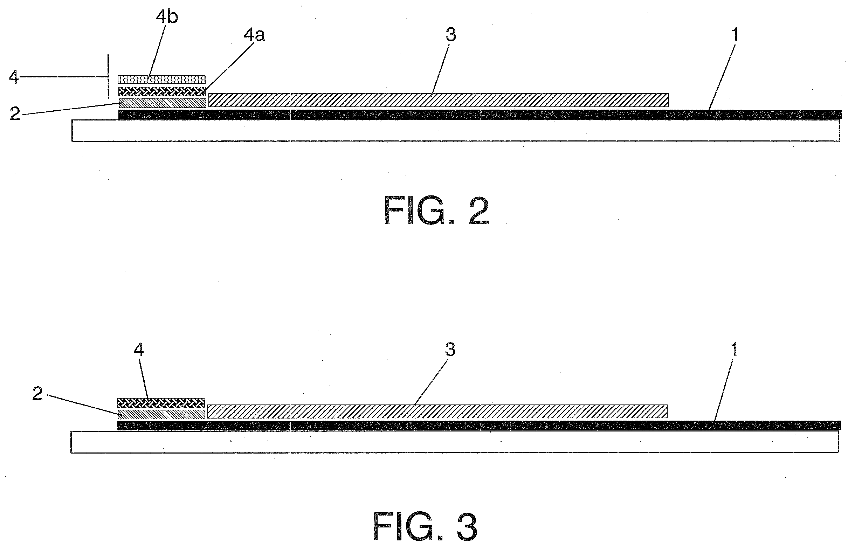

Figure 2 shows a side view of the electrochemical sensor of the invention in which the fourth layer is the layer (d1), which comprises a first layer (4a) comprising a polythiophene deposited on the lower end of the working electrode and a second layer (4b) comprising a non-conductive polymer gel deposited on the layer of polythiophene.
Figure 3 shows a side view of the electrochemical sensor of the invention in which the fourth layer is the layer (d2), which is a layer of conductive polymer gel deposited only on the lower end of the working electrode and comprising a non-conductive polymer gel and a polythiophene.

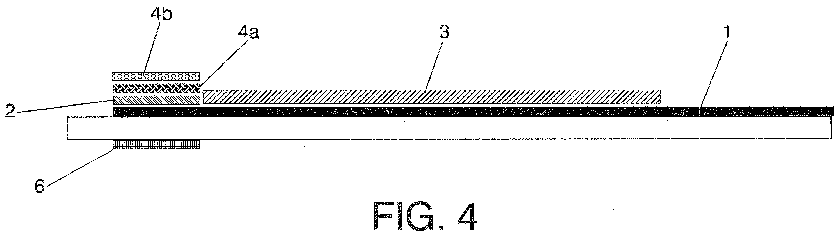

Figure 4 shows a side view of the electrochemical sensor of the present invention in which the fourth layer is the layer (d3), which is a first layer (4a) comprising a polythiophene deposited on the lower end of the working electrode and a second layer (4b) comprising magnetic nanoparticles, functionalized with a biological compound, deposited on said layer of polythiophene, and wherein (6) is a magnet located below the substrate.

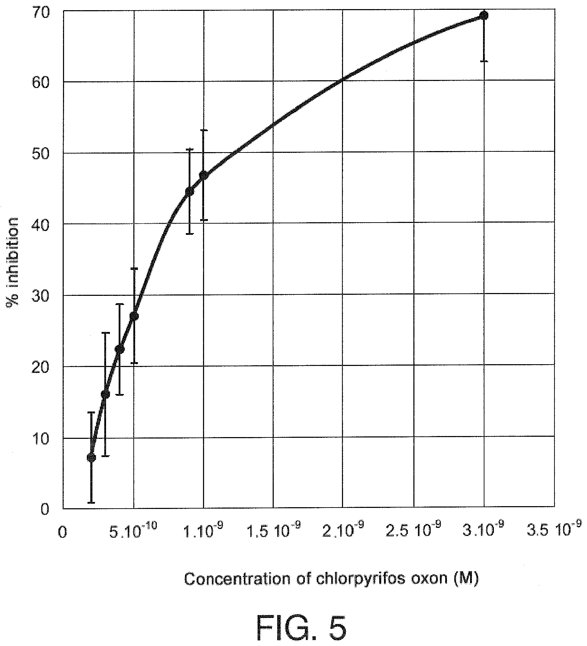

Figure 5 shows the percentage of inhibition of the acetylcholinesterase enzyme as a function of the concentration of the pesticide chlorpyrifos oxon determined in an electrochemical sensor of the invention comprising a first layer of graphite and a fourth layer comprising, in turn, a layer of PEDOT on which a layer of PVA (layer (d1)) is deposited.

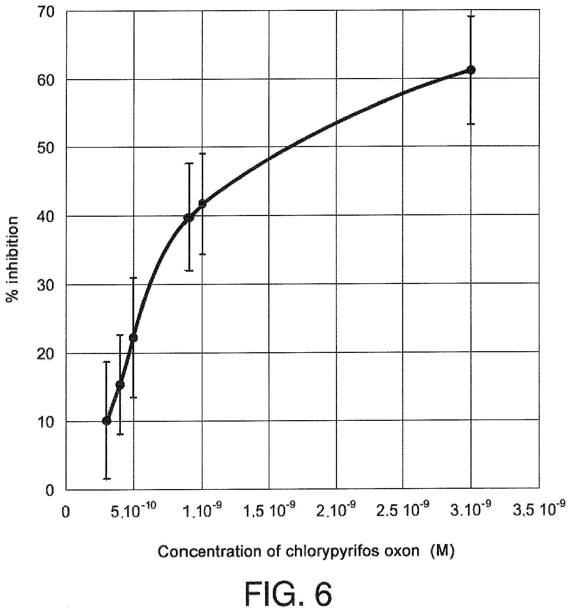

Figure 6 shows the percentage of inhibition of the acetylcholinesterase enzyme as a function of the concentration of the pesticide chlorpyrifos oxon determined in an electrochemical sensor of the invention comprising a first layer of graphite and a fourth layer of PEDOT and PVA (layer (d2)).

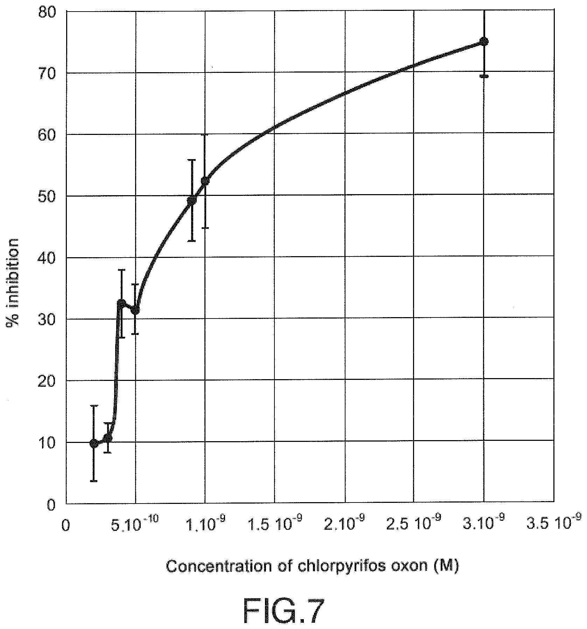

Figure 7 shows the percentage of inhibition of the acetylcholinesterase enzyme as a function of the concentration of the pesticide chlorpyrifos oxon determined in an electrochemical sensor of the invention comprising a first layer of graphite and an intermediate layer of cobalt phthalocyanine on which the fourth layer of PEDOT and PVA (layer (d2)) is deposited.

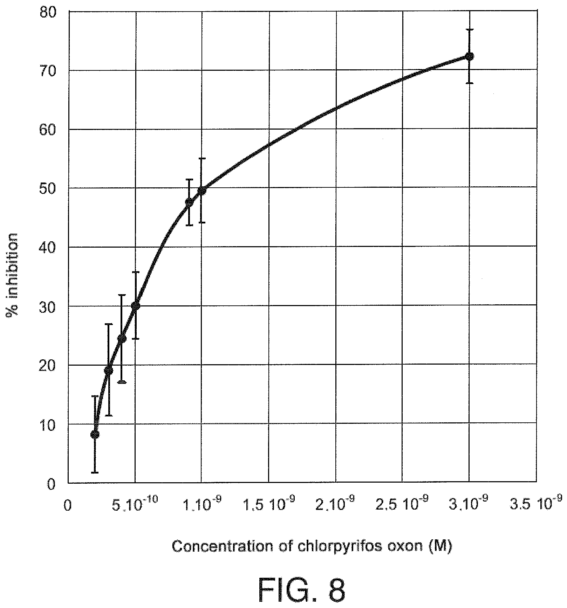

Figure 8 shows the percentage of inhibition of the acetylcholinesterase enzyme as a function of the concentration of the pesticide chlorpyrifos oxon determined in an electrochemical sensor of the invention comprising an intermediate layer of silver deposited on the substrate and on which there is deposited a first layer of graphite and a fourth layer comprising, in turn, a layer of PEDOT on which a layer of PVA (layer (d1)) is deposited.

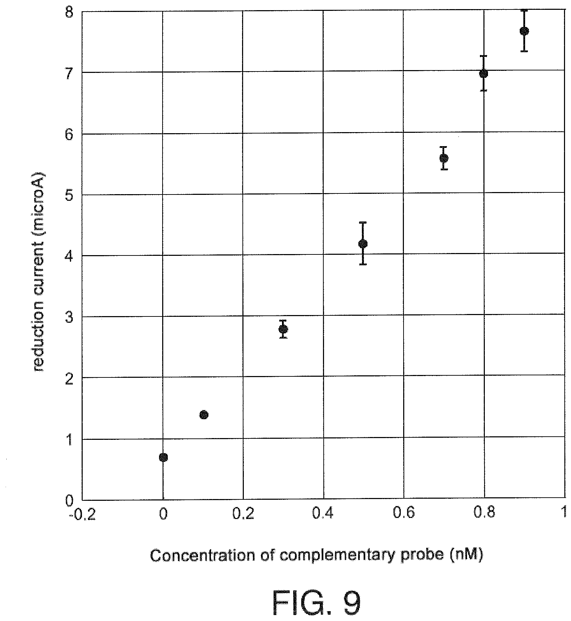

Figure 9 shows the reduction current as a function of the concentration of DNA specific probe determined in an electrochemical sensor of the present invention comprising a magnet below the substrate, a first layer of graphite, and a fourth layer comprising, in turn, a layer of PEDOT on which a layer of gold-coated magnetic nanoparticles functionalized with thiolated probe (layer (d3)) is deposited.

### Detailed Description of the Invention

The present invention provides an electrochemical sensor for the detection of analytes in liquid media, hereinafter "electrochemical sensor of the invention", which comprises:
(a) a first layer (1) comprising a carbonaceous material deposited on a substrate, said layer forming the system of electrodes of the electrochemical sensor which is composed of at least by a pseudo-reference electrode, a working electrode and a counter electrode;
(b) a second layer (2) comprising a metal material deposited only on the lower end of the pseudo-reference electrode;
(c) a third layer (3) comprising an insulating material deposited on a part of the system of electrodes, said part being the one located between the analysis surface (3a) and the electrical contacts (3b) of the measuring equipment, such that only the lower part of the electrodes of the system of electrodes is exposed; and
(d) a fourth layer (4) comprising polythiophene, deposited only on the lower end of the working electrode selected from (d1),(d2) and (d3), wherein:
   (d1) comprises a layer comprising a polythiophene deposited on the lower end of the working electrode and a layer comprising a non-conductive polymer gel deposited on said layer of polythiophene;
   (d2) is a layer of conductive polymer gel comprising a polythiophene and a non-conductive polymer gel; and
   (d3) comprises a layer comprising a polythiophene deposited on the lower end of the working electrode and a layer comprising magnetic nanoparticles, functionalized with a biological compound covalently bound on their surface, deposited on said layer of polythiophene.

This multilayer structure of the electrochemical sensor of the invention can be seen in Figure 1, which depicts the optimal geometry thereof, although it could have other possible geometries.

In the context of the invention, the term "pseudo-reference electrode" relates to an electrode having a stable and known equilibrium potential and which is used to measure the potential against other electrodes in an electrochemical system.

Likewise, in the context of the invention, the term "working electrode" relates to the electrode in which, in an electrochemical system, the reaction of interest occurs. The working electrode is often used in combination with a counter electrode and a reference electrode in a system of three electrodes, although there can be more than one working electrode, giving rise to a system of electrodes or multielectrode system. Depending on whether the reaction in the electrode is a reduction or an oxidation, the working electrode can be considered a cathode or an anode.

Likewise, in the context of the invention, the term "counter electrode" relates to a non-polarizable electrode completing the cell circuit. In laboratory cells, the counter electrode is generally an inert conductor such as platinum or graphite.

Thus, in a particular embodiment, the system of electrodes of the electrochemical sensor of the invention is formed by a pseudo-reference electrode, a working electrode and a counter electrode. In another particular embodiment, the system of electrodes of the electrochemical sensor of the invention is formed by a pseudo-reference electrode, two or more working electrodes and a counter electrode.

In the context of the invention, the expression "detection of analytes in liquid media" relates to both the qualitative determination and the quantitative determination of an analyte contained in a liquid medium (solution, dispersion, etc.) subjected to testing.

Said determination is performed either by immersing the electrochemical sensor of the invention in the liquid medium containing the analyte or by depositing a drop of said liquid medium on the analysis surface (3a) of the electrochemical sensor of the invention.

The substrate can be any suitable substrate known by the person skilled in the art. Thus, in a particular embodiment of the electrochemical sensor of the invention, the substrate is a plastic, textile or paper sheet. In a preferred embodiment, the plastic sheet is formed by polymers with a high melting point or high glass transition temperature, preferably poly(ethylene terephthalate) or poly(carbonate). In another preferred embodiment, the plastic sheet is formed by plasticized poly(vinyl chloride), thermoplastic rubbers, fibers or polymer fabrics.

The first layer (1) comprising a carbonaceous material is deposited on the substrate such that it delimits the geometry of the system of electrodes, as has been indicated above. In a particular embodiment of the electrochemical sensor of the invention, the carbonaceous material of the first layer is selected from graphite, carbon black and carbon nanotubes. In a preferred embodiment, the carbonaceous material is graphite. Thus, the latter can be a graphite paste or ink, i.e., a graphite dispersion.

In a particular embodiment of the electrochemical sensor of the invention, the latter comprises an intermediate layer which comprises a metal material and which is deposited on the substrate before depositing the first layer of carbonaceous material. Said metal material is any suitable metal material of the state of the art. Thus, in a particular embodiment of the electrochemical sensor of the invention, the metal material of this optional intermediate layer is selected from silver, gold, platinum, palladium, copper and tungsten. In a preferred embodiment, the metal material of this optional intermediate layer is silver. In this case, this intermediate layer will delimit the geometry of the system of electrodes.

The second layer (2) is deposited only on the lower end of the reference electrode and can comprise any suitable metal material selected by the person skilled in the art. Thus, in a particular embodiment of the electrochemical sensor of the invention, the metal material is Ag/AgCl (silver/silver chloride) or Hg/Hg₂Cl₂ (calomel). In a preferred embodiment, the metal material is Ag/AgCl.

To protect the layer of carbonaceous material from the environment and delimit the area of exposure to the sample containing the analyte, a third layer of insulating material is used. The third layer (3) is therefore deposited on a part of the system of electrodes, said part being the one located between the analysis surface (3a) and the electrical contacts (3b) of the measuring equipment, such that only the lower part of the electrodes of the system of electrodes is exposed. The measuring equipment used can be any suitable measuring equipment of the state of the art such as a potentiostat, for example.

This third layer (3) comprises any suitable insulating material of the state of the art such as a silicone, an epoxy resin, an acrylic paint or vinyl paint, for example.

The fourth layer (4) comprising a polythiophene is deposited only on the lower end of the working electrode and is selected from three possible layers, (d1), (d2) and (d3):
the layer (d1) comprises a first layer comprising a polythiophene deposited on the lower end of the working electrode and a second layer comprising a non-conductive polymer gel deposited on said layer of polythiophene;
the layer (d2) corresponds to a single layer of conductive polymer gel comprising a non-conductive polymer gel and a polythiophene, and the layer (d3) comprises a layer comprising a polythiophene deposited on the lower end of the working electrode and a layer comprising functionalized magnetic nanoparticles deposited on said layer of polythiophene.

In a particular embodiment of the electrochemical sensor of the invention, the fourth layer (4) comprising polythiophene and deposited only on the lower end of the working electrode is the layer (d1) which comprises a first layer (4a) comprising a polythiophene deposited on the lower end of the working electrode and a second layer (4b) comprising a non-conductive polymer gel deposited on said layer of polythiophene (Figure 2).

The polymer gel of the layer (d1) comprises the biological compound acetylcholinesterase.

In another particular embodiment of the electrochemical sensor of the invention, the fourth layer (4) comprising polythiophene and deposited only on the lower end of the working electrode is the layer (d2) which is a layer of conductive polymer gel comprising a non-conductive polymer gel and a polythiophene (Figure 3). This layer has a conductive nature due to the fact that it contains a conductive polymer such as polythiophene.

The polymer gel of the layer (d2) comprises the biological compound acetylcholinesterase.

In a preferred embodiment, the sensor comprises an additional intermediate layer comprising an electrochemical mediator deposited only on the lower end of the working electrode and on which the layer (d2) of conductive polymer gel is deposited. In this case, the polythiophene of the conductive polymer gel will act mainly as a conductor. The electrochemical mediator used in this optional intermediate layer can be any suitable mediator of the state of the art. Thus, in an even more preferred embodiment, the electrochemical mediator is selected from cobalt phthalocyanine (CoPh), 7,7,8,8-tetracyanoquinodimethane (TCNQ), hydroquinone (HQ), quinone (Q), tetrathiafulvalene (TTF) and ferrocene (FC). In a much more preferred embodiment, the electrochemical mediator is cobalt phthalocyanine (CoPh).

In another particular embodiment of the electrochemical sensor of the invention, the fourth layer (4) is the layer (d3) which comprises a layer (4a) comprising a polythiophene deposited on the lower end of the working electrode and a layer (4b) comprising magnetic nanoparticles, functionalized with the biological compound acetylcholinesterase covalently bound on their surface, deposited on said layer of polythiophene (Figure 4).

In this particular embodiment, and preferably, the electrochemical sensor has a magnet (6) coupled below the substrate. The functionalized magnetic nanoparticles are thus captured on the layer of polythiophene due to the effect of the magnet, thus moving closer to the working electrode for the purpose of performing the detection. The material of the magnet can be of any magnetic material (neodymium, iron, cobalt, nickel, magnetite, copper/nickel/cobalt alloys, iron/cobalt/vanadium alloys, etc).

In the context of the present invention, the functionalized magnetic nanoparticles of the layer d3 are nanometric-sized particles which can be handled by means of a magnetic field, which are formed by magnetic elements such as iron, nickel, copper, cobalt, or chemical derivatives of these elements.

The biological compound acetylcholinesterase covalently bound on the surface of the magnetic nanoparticles of the layer (d3) or incorporated in the polymer gel of the layers (d1) or (d2) reacts specifically with certain analytes, allowing their quantification by means of an electrochemical signal.

In a particular embodiment of the electrochemical sensor of the invention, the polythiophene of the fourth layer (4) contains repetitive structural units of formula (I), 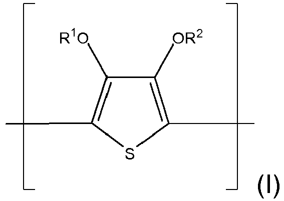 wherein R¹ and R² are independently a C₁-C₁₂ alkyl group or form a C₁-C₁₂ 1,n-alkylene group, with n = 1-12, optionally substituted by a C₁-C₁₂ alkyl, C₂-C₁₂ alkene, vinylene, benzyl, phenyl, halogen group, or by an ester, amino, amido or ether functional group optionally substituted by a C₁-C₁₂ alkyl group.

In a preferred embodiment, in the polythiophene of formula (I) the R¹ and R² groups form an alkylene group selected from methylene, 1,2-ethylene and 1,3-propylene. In an even more preferred embodiment, in the polythiophene of formula (I) the R¹ and R² groups form a 1,2-ethylene group.

Said polythiophenes in their oxidized state can additionally incorporate anionic groups, stabilizing the delocalized positive type charge carriers in the polymer chains. Thus, in another particular embodiment of the electrochemical sensor of the invention, the polythiophene of the fourth layer comprises an anionic dopant. In a preferred embodiment, the anionic dopant is an inorganic anion selected from a sulfate, chloride and bromide anion. In another preferred embodiment, the anionic dopant is an organic anion with sulfonate or phosphate groups selected from a p-toluenesulfonic acid and a p-toluenephosphonic acid. In another preferred embodiment, the anionic dopant is an organic polyanion selected from polymeric carboxylic acids, polymeric sulfonic acids, or copolymers of vinycarboxylic acids and vinylsulfonic acids with other polymerizable monomers. In an even more preferred embodiment, the anionic dopant is an organic polyanion selected from poly(acrylic acid), poly(methacrylic acid) and poly(maleic acid). In another even more preferred embodiment, the anionic dopant is an organic polyanion selected from poly(styrene sulfonic) acid or poly(vinylsulfonic) acid. In another even more preferred embodiment, the anionic dopant is an organic polyanion selected from copolymers of vinycarboxylic acids and vinylsulfonic acids with styrene and acrylic or methacrylic monomers. In another even more preferred embodiment, the anionic dopant is an organic polyanion the molecular weight of which is comprised between 15,000 and 300,000 Daltons.

The non-conductive polymer gel of the fourth layer (4)(d1 or d2) will be any non-conductive polymer gel of the state of the art which absorbs in its interior the solution containing the analyte, preferably a crosslinked polymer hydrogel. Thus, in a particular embodiment of the electrochemical sensor of the invention, the non-conductive polymer gel is selected from among poly(vinyl alcohol), glutaraldehyde, hydroxyethylcellulose, polymethylmethacrylate derivatives, polyethylene glycol derivatives and Nafion. In a preferred embodiment, the non-conductive polymer gel is photocrosslinkable poly(vinyl alcohol).

In another main aspect of the invention, there is provided a process for preparing the electrochemical sensor of the invention, hereinafter "the process of the invention", which comprises:
(A) obtaining on the substrate the first layer comprising a carbonaceous material and forming the system of electrodes formed at least by a pseudo-reference electrode, a working electrode and a counter electrode;
(B) obtaining the second layer comprising a metal material only on the lower end of the pseudo-reference electrode;
(C) obtaining the third layer comprising insulating material on the part of the system of electrodes located between the analysis surface and the electrical contacts of the measuring equipment such that it leaves only the lower part of the electrodes of the system of electrodes exposed; and
(D) obtaining the fourth layer comprising polythiophene only on the lower end of the working electrode.

The fourth layer comprising polythiophene is obtained by means of a method selected from (D1), (D2) and (D3), wherein:
(D1) comprises obtaining the layer comprising a polythiophene on the lower end of the working electrode and then obtaining the layer comprising a polymer gel on the layer comprising a polythiophene;
(D2) comprises obtaining the layer comprising a polymer gel and a polythiophene on the lower end of the working electrode, and
(D3) comprises obtaining the layer comprising a polythiophene on the lower end of the working electrode and then obtaining the layer comprising the magnetic nanoparticles, functionalized with the biological compound acetylcholinesterase covalently bound on their surface, on the layer comprising a polythiophene.

In a particular embodiment, the fourth layer is obtained by means of method D1 or D2. In this case, the process of the invention comprises an additional stage which comprises incorporating the biological compound acetylcholinesterase in the polymer gel of d1 or d2, respectively.

In another particular embodiment, the fourth layer is obtained by means of method D3. In this case, an additional stage before stage D3 which comprises coupling a magnet below the substrate is contemplated.

Obtaining the fourth layer comprises:
(i) the application of either aqueous or solvent-based true solutions, colloidal dispersions or stable dispersions of finely divided particles of polythiophene previously obtained by means of oxidative polymerization or enzymatic polymerization; or
(ii) the application of either aqueous or solvent-based solutions of thiophene monomers and subsequent *in situ* polymerization thereof.

In particular, with respect to method (D1), an either aqueous or solvent-based true solution, colloidal dispersion or stable dispersion of finely divided particles of a polythiophene previously obtained by means of oxidative polymerization or enzymatic polymerization is applied on the lower end of the working electrode. Said application is performed by means of different known techniques such as painting, immersion, spin coating or screen printing, for example. After the application of said solution or dispersion of polythiophene the direct evaporation of the solvent is carried out. In another variant, a solution of thiophene monomers is applied on the lower end of the working electrode in a manner similar to that described above and then the *in situ* polymerization of said monomers and the subsequent evaporation of the solvent are carried out.

Then, and in a similar manner, a solution of prepolymer of a non-conductive polymer gel is manually applied on the layer of polythiophene thus obtained. After the application of said solution, the prepolymer is crosslinked by means of any known technique such as exposure to halogen light, for example, and then the direct evaporation of the solvent is carried out.
On the other hand, with respect to method (D2), an either aqueous or solvent-based true solution, colloidal dispersion or stable dispersion of finely divided particles of polythiophene mixed with a solution of the non-conductive polymer gel is applied on the lower end of the working electrode. After the application of said mixture, the direct evaporation of the solvent is carried out.

As an alternative, a solution of the non-conductive polymer gel comprising the thiophene monomers is applied on the lower end of the working electrode. Said application is performed manually. After the application of said solution, the thiophene monomers are polymerized inside the non-conductive polymer gel by means of *in situ* polymerization (oxidative polymerization or enzymatic polymerization) and the prepolymer is subsequently crosslinked by means of any known technique such as exposure to halogen light, for example. Finally, the direct evaporation of the solvent is carried out.

With respect to method (D3), a solution containing the functionalized magnetic nanoparticles in suspension is manually applied on the layer of polythiophene obtained by means of the same process used in method (D1). Said particles agglutinate and are deposited on the working electrode due to the magnetic attraction exerted on them by the magnet.

In any case, in the process of the invention the polythiophene is chemically synthesized, which is a much simpler and less expensive method than the electrochemical synthesis thereof.

These methods of oxidative polymerization, enzymatic polymerization or *in situ* polymerization of the corresponding monomer can be those described in the references ADVANCED FUNCTIONAL MATERIALS 14, 615-622, 2004 and BIOMACROMOLECULES 8(2), 315-317, 2007. Preferable solvents include alcohols (methanol, ethanol or isopropanol, for example), as well mixtures of water with these alcohols or other water-miscible organic solvents such as acetone, for example. For the oxidative polymerization, ammonium persulfate, iron trichloride or ferric tosylate can be used as preferred oxidizers. For the enzymatic polymerization, horseradish peroxidase or peroxidases of other origins can be used as preferred enzymes. Additionally, polymeric binders of the type of poly(vinyl alcohol), poly(vinyl acetate), etc., and adhesion promoters, of the type of silanes, tackifying resins, etc., to facilitate the formation of films highly adherent on the corresponding, can be used.

In a similar manner, the rest of the layers comprised in the electrochemical sensor of the invention can be obtained by applying the corresponding dispersion or solution on the previous layer by means of different known techniques such as painting, immersion, spin coating or screen printing, for example, followed by the direct evaporation of the solvent.

In a particular embodiment of the process of the invention, the latter comprises obtaining an intermediate layer comprising a metal material on the substrate before obtaining the first layer.

In another particular embodiment of the process of the invention, the latter comprises obtaining an intermediate layer comprising an electrochemical mediator on the lower end of the working electrode before obtaining the layer (d2) of conductive polymer gel, as has been indicated above.

These optional intermediate layers can also be obtained by applying the corresponding dispersion on the previous layer by means of the different mentioned techniques, followed by the direct evaporation of the solvent.

The electrochemical sensor of the invention can be used for the detection of analytes of a different nature such as, for example, pesticides (organophosphates and carbamates, for example), pathogens, heavy metals, neurotransmitters, metabolites, nucleotides, oligonucleotides, polynucleotides (DNA, RNA) etc.

The following examples illustrate the invention and must not be considered as limiting the scope thereof.

### Example 1

### Preparation of an electrochemical sensor for the detection of the pesticide chlorpyrifos oxon comprising a first layer of graphite and a fourth layer comprising, in turn, a layer of PEDOT on which a layer of PVA (layer (d1)) is deposited.

An electrochemical sensor with three electrodes according to the invention for the detection of chlorpyrifos oxon, an organophosphate pesticide, based on the inhibition of thiocholine production, was prepared according to the following process. 1) 3 tracks of a commercial conductive graphite ink (Electrodag PF-410) were printed by means of screen printing on a plastic support of polycarbonate PC. 2) A layer of a commercial Ag/AgCl ink (Electromag. 6037 SS) was printed by means of screen printing only on the lower end of the reference electrode. 3) A protective layer of commercial vinyl paint (Electrodag 452 SS) was printed by means of screen printing on part of the 3 electrodes, leaving only the lower part of the working electrode, of the reference electrode and of the counter electrode exposed. 4a) A layer of an aqueous dispersion of polyethylenedioxythiophene (PEDOT) at 1% by weight of polythiophene (previously prepared from an aqueous solution of 0.1 M commercial ethylenedioxythiophene, EDOT, monomer (99%, Sigma-Aldrich Chemicals), and 0.1 M commercial polystyrene sulfonate, PSS, (Sigma-Aldrich Chemicals), as a dopant-stabilizer at room temperature, which was vigorously stirred, the oxidizer ammonium persulfate (0.1 M) was added thereto and it was left to polymerize) was printed by means of screen printing on the lower end of the working electrode. 4b) A mixture of aqueous solution of commercial photocrosslinkable polyvinyl alcohol (PVA) (Aldrich) at 6% by weight and a solution of commercial acetylcholinesterase enzyme (from electric eel, type V-S, of Sigma) in phosphate buffer was manually deposited on the layer of PEDOT. The activity of the enzyme solution was between 0.07 and 0.18 AU/min according to the PVA/enzyme ratio of the mixture (30/70, 50/50 or 70/30). In any of the cases, 1 enzyme mU (amount necessary for catalyzing the conversion of 1 µmol of substrate per minute) was immobilized in the working electrode. The enzyme was trapped in the working electrode after crosslinking the prepolymer by means of exposure to Neon or halogen light, of λ>400 nm, for 24-72 hours, depending on the amount of PVA in the PVA/enzyme mixture.

The sensor thus obtained has the layered structure defined in Figure 1 (top view) and in Figure 2 (side view), with the following areas of the electrodes:
Area of the working electrode: 58.09 mm²
Area of the reference electrode: 3.74 mm²
Area of the counter electrode: 7.9 mm²
and the following ratios between said areas:
Area of the reference electrode/Area of the working electrode = 0.064 mm²
Area of the counter electrode /Area of the working electrode = 0.136 mm²

After obtaining the sensor, the concentration of chlorpyrifos oxon was determined in an aqueous solution. To that end, acetylcholine chloride (a drop of 30 µl, 50 mM) was deposited on the analysis surface of the sensor, whereby thiocholine was enzymatically produced. The oxidation of this thiocholine was determined in the sensor at a potential of 100 mV, with the compound polythiophene as a mediator. The concentration of acetylthiocholine chloride necessary for obtaining a maximum current intensity signal in the sensor is defined as the "saturation concentration". Taking the saturation conditions as a reference, in the presence of the pesticide chlorpyrifos oxon, the current intensity signal obtained is lower due to the enzyme deactivation caused by the pesticide in the sensor. This decrease of the signal in the presence of different concentrations of pesticide enables the calibration of the sensor, and subsequently its use in the quantification of the pesticide in the solution to be tested.

The percentage of inhibition of the acetylcholinesterase enzyme in the presence of different concentrations of chlorpyrifos oxon was thus determined. The results obtained are shown in Table 1 and in Figure 5.

**Table 1 - Percentage of inhibition of the acetylcholinesterase enzyme in the presence of different concentrations of chlorpyrifos oxon**

| Concentration of pesticide Chlorpyrifos oxon (M) | % of inhibition in the presence of pesticide | Standard deviation | No. of measurements the average of which has been calculated |
|---|---|---|---|
| 2.10⁻¹⁰ | 7.21 | 6.30 | 8 |
| 3.10⁻¹⁰ | 16.11 | 8.64 | 8 |
| 4.10⁻¹⁰ | 22.41 | 6.35 | 8 |
| 5.10⁻¹⁰ | 27.09 | 6.61 | 6 |
| 9.10⁻¹⁰ | 44.54 | 5.94 | 6 |
| 1.10⁻⁹ | 46.82 | 6.33 | 7 |
| 3.10⁻⁹ | 69.20 | 6.51 | 7 |

As can be seen, the limits of detection are very low, since it is possible to detect 2.10⁻¹⁰ M of pesticide with a 7% inhibition.

### Example 2

### Preparation of an electrochemical sensor for the detection of the pesticide chlorpyrifos oxon comprising a first layer of graphite and a fourth layer of PEDOT and PVA (layer (d2)).

An electrochemical sensor with three electrodes according to the invention for the detection of chlorpyrifos oxon, an organophosphate pesticide, based on the inhibition of thiocholine production, was prepared according to the following process. The first 3 layers were deposited in a manner identical to the description provided in Example 1. Then, the following was carried out. 4) An aqueous solution of commercial photocrosslinkable polyvinyl alcohol (Aldrich) at 6% by weight, containing the commercial acetylcholinesterase enzyme (from electric eel, type V-S, of Sigma) with a defined activity therein (between 0.07 and 0.18 AU/min according to the PVA/enzyme ratio of the mixture (30/70, 50/50 or 70/30)), and a solution of 0.1 M commercial ethylenedioxythiophene, EDOT, monomer (99%, Sigma-Aldrich Chemicals) was manually deposited on the lower end of the working electrode. The monomer was made to polymerize by means of enzymatic polymerization using the horseradish peroxidase enzyme (type VI HRP of Sigma)/H₂O₂ (0.3 mg/ml) and, finally, the PVA prepolymer was crosslinked by means of exposure to Neon or halogen light, of λ>400 nm, for 24-72 hours, depending on the amount of PVA in the PVA/enzyme mixture, the enzyme finally being trapped inside a conductive polymer gel.

The sensor thus obtained has the layered structure defined in Figure 1 (top view) and in Figure 3 (side view), with the areas and area ratios of Example 1.

After obtaining the sensor of the invention, the concentration of chlorpyrifos oxon was determined in an aqueous solution. To that end, acetylcholine chloride (a drop of 30 µl, 50 mM) was deposited on the analysis surface of the sensor, whereby thiocholine was enzymatically produced. The oxidation of this thiocholine was determined in the sensor at a potential of 100 mV, with the compound polythiophene as a mediator. The concentration of acetylthiocholine chloride necessary for obtaining a maximum current intensity signal in the sensor is defined as the "saturation concentration". Taking the saturation conditions as a reference, in the presence of the pesticide chlorpyrifos oxon, the current intensity signal obtained is lower due to the enzyme deactivation caused by the pesticide in the sensor. This decrease of the signal in the presence of different concentrations of pesticide enables the calibration of the sensor, and subsequently its use in the quantification of the pesticide in the solution to be tested.

The percentage of inhibition of the acetylcholinesterase enzyme in the presence of different concentrations of chlorpyrifos oxon was thus determined. The results obtained are shown in Table 2 and in Figure 6.

**Table 2 - Percentage of inhibition of the acetylcholinesterase enzyme in the presence of different concentrations of chlorpyrifos oxon**

| Concentration of pesticide Chlorpyrifos oxon (M) | % of inhibition in the presence of pesticide | Standard deviation | Number of measurements the average of which has been calculated |
|---|---|---|---|
| 2.10⁻¹⁰ | - | - | - |
| 3.10⁻¹⁰ | 10.09 | 8.55 | 8 |
| 4.10⁻¹⁰ | 15.31 | 7.24 | 8 |
| 5.10⁻¹⁰ | 22.19 | 8.72 | 6 |
| 9.10⁻¹⁰ | 39.75 | 7.76 | 6 |
| 1.10⁻⁹ | 41.71 | 7.34 | 7 |
| 3.10⁻⁹ | 61.15 | 7.93 | 7 |

As can be seen, the limits of detection are very low, since it is possible to detect 3.10⁻¹⁰ M of pesticide with a 10% inhibition.

### Example 3

### Preparation of an electrochemical sensor for the detection of the pesticide chlorpyrifos oxon comprising a first layer of graphite and an intermediate layer of cobalt phthalocyanine on which the fourth layer of PEDOT and PVA (layer (d2)) is deposited.

An electrochemical sensor with three electrodes according to the invention for the detection of chlorpyrifos oxon, an organophosphate pesticide, based on the inhibition of thiocholine production, was prepared according to the following process. The first 3 layers were deposited in a manner identical to the description made in Example 1. Then, the following was carried out. 3'). A layer of a dispersion (3.8 mg/ml) of cobalt phthalocyanine (CoPh) obtained by means of the solution of commercial CoPh (Sigma) in an aqueous solution of hydroxyethylcellulose (HEC) at 4% was deposited by means of screen printing on the lower end of the working electrode. 4) An aqueous solution of commercial photocrosslinkable polyvinyl alcohol (Aldrich) at 6% by weight, containing the commercial acetylcholinesterase enzyme (from electric eel, type V-S, of Sigma) with a defined activity therein (between 0.07 and 0.18 AU/min according to the PVA/enzyme ratio of the mixture (30/70, 50/50 or 70/30)), and a solution of 0.1 M commercial ethylenedioxythiophene, EDOT, monomer (99%, Sigma-Aldrich Chemicals) was manually deposited on the previous layer. The monomer was made to polymerize by means of enzymatic polymerization using the horseradish peroxidase enzyme (type VI HRP of Sigma)/H₂O₂ (0.3 mg/ml) and, finally, the PVA prepolymer was crosslinked by means of exposure to Neon or halogen light, of λ>400 nm, for 24-72 hours, depending on the amount of PVA in the PVA/enzyme mixture, the enzyme finally being trapped inside a conductive polymer gel.

The sensor thus obtained has the layered structure defined in Figure 1 (top view) and in Figure 3 (side view), with the areas and area ratios of Example 1.

After obtaining the sensor of the invention, the concentration of chlorpyrifos oxon was determined in an aqueous solution. To that end, acetylcholine chloride (a drop of 30 µl, 50 mM) was deposited on the analysis surface of the sensor, whereby thiocholine was enzymatically produced. The oxidation of this thiocholine was determined in the sensor at a potential of 100 mV, with the compound polythiophene as a mediator. The concentration of acetylthiocholine chloride necessary for obtaining a maximum current intensity signal in the sensor is defined as the "saturation concentration". Taking the saturation conditions as a reference, in the presence of the pesticide chlorpyrifos oxon, the current intensity signal obtained is lower due to the enzyme deactivation caused by the pesticide in the sensor. This decrease of the signal in the presence of different concentrations of pesticide enables the calibration of the sensor, and subsequently its use in the quantification of the pesticide in the solution to be tested.

The percentage of inhibition of the acetylcholinesterase enzyme in the presence of different concentrations of chlorpyrifos oxon was thus determined. The results obtained are shown in Table 3 and in Figure 7.

**Table 3 - Percentage of inhibition of the acetylcholinesterase enzyme in the presence of different concentrations of chlorpyrifos oxon**

| Concentration of pesticide Chlorpyrifos oxon (M) | % of inhibition in the presence of pesticide | Standard deviation | Number of measurements the average of which has been calculated |
|---|---|---|---|
| 2.10⁻¹⁰ | 9.82 | 6.06 | 8 |
| 3.10⁻¹⁰ | 10.70 | 2.36 | 8 |
| 4.10⁻¹⁰ | 32.51 | 5.43 | 8 |
| 5.10⁻¹⁰ | 31.46 | 3.91 | 6 |
| 9.10⁻¹⁰ | 49.18 | 6.51 | 6 |
| 1.10⁻⁹ | 52.36 | 7.65 | 7 |
| 3.10⁻⁹ | 74.80 | 5.62 | 7 |

As can be seen, the limits of detection are very low, since it is possible to detect 2.10⁻¹⁰ M of pesticide with a 9% inhibition.

### Example 4

### Preparation of an electrochemical sensor for the detection of the pesticide chlorpyrifos oxon comprising an intermediate layer of silver on which a first layer of graphite is deposited and a fourth layer comprising, in turn, a layer of PEDOT on which a layer of PVA (layer (d1)) is deposited.

An electrochemical sensor with three electrodes according to the invention for the detection of chlorpyrifos oxon, an organophosphate pesticide, based on the inhibition of thiocholine production, was prepared according to the following process. 1') Three tracks of a commercial conductive silver ink (Acheson) were printed by means of screen printing on a plastic support of polycarbonate PC. 1) 3 tracks of a commercial conductive graphite ink (Electrodag PF-410) were printed by means of screen printing on the previous layer of silver. Layers 2 and 3 were deposited in a manner identical to Example 1. 4a) A layer of an aqueous dispersion of polyethylenedioxythiophene (PEDOT) at 1% by weight, previously prepared, was printed by means of screen printing on the lower end of the working electrode. To that end, equimolar amounts of ethylenedioxythiophene, EDOT, monomer (0.1 M) and the dopant-stabilizer polystyrene sulfonate, PSS, (0.1 M) were dissolved in water at room temperature and the pH was adjusted to 2 by means of adding HCl; then, 0.3 mg/ml of commercial horseradish peroxidase (type VI HRP of Sigma) and an equimolar amount of H₂O₂ (0.055 M) were added and left to polymerize for 16 hours at 4°C, obtaining a dispersion of approximately 1% PEDOT in water. 4b) A mixture of aqueous solution of commercial photocrosslinkable polyvinyl alcohol (PVA) (Aldrich) at 6% by weight and a solution of commercial acetylcholinesterase enzyme (from electric eel, type V-S, of Sigma) in phosphate buffer was manually deposited on the layer of PEDOT. The activity of the enzyme solution was between 0.07 and 0.18 AU/min according to the PVA/enzyme ratio of the mixture (30/70, 50/50 or 70/30). In any of the cases, 1 enzyme mU (amount necessary for catalyzing the conversion of 1 µmol of substrate per minute) was immobilized in the working electrode. The enzyme was trapped in the working electrode after crosslinking the prepolymer by means of exposure to Neon or halogen light, of λ>400 nm, for 24-72 hours, depending on the amount of PVA in the PVA/enzyme mixture.

The sensor thus obtained has the layered structure defined in Figure 1 (top view) and in Figure 2 (side view), with the areas and area ratios of Example 1.

After obtaining the sensor of the invention, the concentration of chlorpyrifos oxon was determined in an aqueous solution. To that end, acetylcholine chloride (a drop of 30 µl, 50 mM) was deposited on the analysis surface of the sensor, whereby thiocholine was enzymatically produced. The oxidation of this thiocholine was determined in the sensor at a potential of 100 mV, with the compound polythiophene as a mediator. The concentration of acetylthiocholine chloride necessary for obtaining a maximum current intensity signal in the sensor is defined as the "saturation concentration". Taking the saturation conditions as a reference, in the presence of the pesticide chlorpyrifos oxon, the current intensity signal obtained is lower due to the enzyme deactivation caused by the pesticide in the sensor. This decrease of the signal in the presence of different concentrations of pesticide enables the calibration of the sensor, and subsequently its use in the quantification of the pesticide in the solution to be tested.

The percentage of inhibition of the acetylcholinesterase enzyme in the presence of different concentrations of chlorpyrifos oxon was thus determined. The results obtained are shown in Table 4 and in Figure 8.

**Table 4 - Percentage of inhibition of the acetylcholinesterase enzyme in the presence of different concentrations of chlorpyrifos oxon**

| Concentration of pesticide Chlorpyrifos oxon (M) | % of inhibition in the presence of pesticide | Standard deviation | Number of measurements the average of which has been calculated |
|---|---|---|---|
| 2.10⁻¹⁰ | 8.28 | 6.40 | 8 |
| 3.10⁻¹⁰ | 19.14 | 7.67 | 8 |
| 4.10⁻¹⁰ | 24.51 | 7.37 | 8 |
| 5.10⁻¹⁰ | 30.10 | 5.64 | 6 |
| 9.10⁻¹⁰ | 47.55 | 3.84 | 6 |
| 1.10⁻⁹ | 49.50 | 5.43 | 7 |
| 3.10⁻⁹ | 72.23 | 4.62 | 7 |

As can be seen, the limits of detection are very low, since it is possible to detect 2.10⁻¹⁰ M of pesticide with an 8% inhibition.

### Example 5

### Preparation of an electrochemical sensor for the detection of DNA specific sequences comprising a first layer of graphite and a fourth layer comprising, in turn, a layer of PEDOT on which a layer of magnetic nanoparticles (layer (d3)) is deposited.

An electrochemical sensor with three electrodes according to the invention for the detection of specific probes, by means of enzymatic amplification, using hydroquinone as a redox mediator and hydrogen peroxide as an enzymatic substrate, was prepared. This electrochemical sensor is based on using gold-coated magnetic nanoparticles for carrying out the processes for immobilizing the thiolated DNA probe (19 mer), according to the following process. The first 3 layers were deposited in a manner identical to the description made in Example 1. Then, the following was carried out. 4a) A layer of an aqueous dispersion of polyethylenedioxythiophene (PEDOT) at 1% by weight of polythiophene (previously prepared from an aqueous solution of 0.1 M commercial ethylenedioxythiophene, EDOT, monomer (99%, Sigma-Aldrich Chemicals), and 0.1 M commercial polystyrene sulfonate, PSS, (Sigma-Aldrich Chemicals), as a dopant-stabilizer at room temperature, which was vigorously stirred, the oxidizer ammonium persulfate (0.1 M) was added thereto and it was left to polymerize) was printed by means of screen printing on the lower end of the working electrode. The attraction of the magnetic nanoparticles to the electrode surface was carried out by placing a neodymium magnet in the lower part of the system (6), and 4b) a drop of 30 µl of the solution of substrate (hydrogen peroxide) was deposited on the surface of the electrode in which the mediator and the functionalized magnetic nanoparticles were previously located.

The functionalization of the magnetic nanoparticles was performed as follows. 15 µL of gold-coated magnetic nanoparticles were taken and placed in a 1.5 mL microcentrifuge tube, they were washed twice with 90 µL of 0.1 M phosphate buffer (PBS), pH 7.2, and resuspended in 40 µL of the same buffer which contained 1.03 µmol of thiolated probe. The reaction was left overnight at room temperature at 600 rpm to allow the binding of the thiolated probe to the gold-coated magnetic nanoparticles. After this time had elapsed, they were washed twice with 100 µL of PBS and resuspended again in 90 µL of the same buffer with the desired amount of complementary biotinylated probe and left to react for 1 h at room temperature with constant stirring (600 rpm). The magnetic nanoparticles derivatized with the hybrid were washed twice with the 0.1 M PBS buffer of pH 7.2.

50 µL of HRP-streptavidin (10 µg mL⁻¹), prepared in 0.01 M phosphate buffered saline, of pH 7.0, with 0.01% BSA (PBSB), were then added and left to react for 30 minutes at room temperature. After the reaction time had elapsed, the nanoparticles were washed 5 times for 5 minutes with PBSB.

The sensor thus obtained has the layered structure defined in Figure 1 (top view) and in Figure 4 (side view).
After depositing the drop of analyte on the analysis surface of the sensor and obtaining the sensor of the invention, the concentration of DNA specific sequence was determined. To that end, the enzyme previously bound in the complementary probe was oxidized by means of squarewave voltammetry (SWV), sweeping the potential between 0.3 and -0.4V, using hydroquinone as a mediator and hydrogen peroxide as a substrate of the enzymatic reaction of the peroxidase. The use of hydroquinone as a mediator increases the electron transfer between the peroxidase and the electrode surface.
The reduction current generated in SWV is directly proportional to the amount of enzyme conjugate and, therefore, to the amount of hybridized complementary probe in the functionalized magnetic nanoparticles modified with the probe. Thus, this reduction current generated in SWV in the presence of different concentrations of complementary probe enables the calibration of the sensor, and subsequently, its quantification of specific complementary probe in the solution to be tested.
Different concentrations of specific complementary probe were thus determined. The results obtained are shown in Table 5 and in Figure 9.

**Table 5 - Reduction current generated in the presence of different concentrations of specific complementary probe**

| Concentration of specific complementary probe (nM) | Reduction current (µA) | Standard deviation (%) | Number of measurements the average of which has been calculated |
|---|---|---|---|
| 0.0 | 0.688 | 4.3 | 5 |
| 0.1 | 1.384 | 2.7 | 5 |
| 0.3 | 2.776 | 5 | 5 |
| 0.5 | 4.168 | 8 | 5 |
| 0.7 | 5.56 | 3.1 | 5 |
| 0.8 | 6.952 | 4.0 | 5 |
| 0.9 | 7.648 | 4.3 | 5 |

The sensor thus obtained has the following analytical characteristics:

| Analytical characteristics | |
|---|---|
| Linear range (M) | 0 - 1.1×10⁻⁹ |
| Slope (A µM⁻¹) | (6.96±0.10)×10⁻³ |
| Linear regression coefficient | 0.993 |
| Intercept (µA) | (6.88±1.20) |
| Limit of detection (pM) | 31 |
| Limit of determination (pM) | 104 |

As can be seen, the limits of detection are very low, since it is possible to detect 3.10⁻¹¹ M of specific complementary probe.

## Claims

1. Electrochemical sensor for the detection of analytes in liquid media which comprises:
(a) a first layer (1) consisting on a carbonaceous material printed on a substrate, said layer forming the system of electrodes of the electrochemical sensor which is composed of at least by a pseudo-reference electrode, a working electrode and a counter electrode; wherein the carbonaceous material delimits the geometry of the system of electrodes;
(b) a second layer (2) comprising a metal material printed only on the lower end of the pseudo-reference electrode;
(c) a third layer (3) comprising an insulating material printed on a part of the system of electrodes, said part being the one located between the analysis surface (3a) and the electrical contacts (3b) of the measuring equipment, such that only the lower part of the electrodes of the system of electrodes is exposed; and
(d) a fourth layer (4) comprising polythiophene, previously obtained by means of oxidative polymerization, which simultaneously acts as a mediator and conductor, printed only on the lower end of the working electrode selected from (d1), (d2) and (d3), wherein:
d1) comprises a layer comprising a polythiophene printed on the lower end of the working electrode and a layer comprising a non-conductive polymer gel deposited on said layer of polythiophene, wherein the polymer gel comprises acetylcholinesterase;
d2) is a layer of conductive polymer gel consisting on a polythiophene and a non-conductive polymer gel, wherein the polymer gel comprises acetylcholinesterase; and
d3) comprises a layer comprising a polythiophene printed on the lower end of the working electrode and a layer comprising magnetic nanoparticles, functionalized with a acetylcholinesterase covalently bound on their surface, deposited on said layer of polythiophene.

2. Sensor according to claim 1, **characterized in that**, when the fourth layer (4) is the layer (d3), it may additionally comprises a magnet coupled below the substrate.

3. Sensor according to any one of claims 1 and 2, **characterized in that** the polythiophene contains repetitive structural units of formula (I), 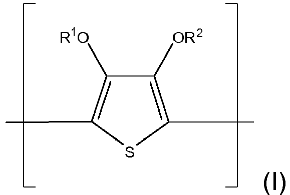 wherein R¹ and R² are independently a C₁-C₁₂ alkyl group or form a C₁-C₁₂ 1,n-alkylene group, with n = 1-12, optionally substituted by a C₁-C₁₂ alkyl, C₂-C₁₂ alkene, vinylene, benzyl, phenyl, halogen group, or by an ester, amino, amido or ether functional group optionally substituted by a C1-C12 alkyl group.

4. Sensor according to claim 3, **characterized in that** in the polythiophene the R¹ and R² groups form an alkylene group selected from methylene, 1,2-ethylene and 1,3-propylene.

5. Sensor according to claim 3, **characterized in that** the polythiophene comprises an anionic dopant.

6. Sensor according to claim 5, **characterized in that** the anionic dopant is an inorganic anion selected from a sulfate, chloride and bromide anion; an organic anion with sulfonate or phosphate groups selected from a p-toluenesulfonic acid and a p-toluenephosphonic acid; or an organic polyanion selected from polymeric carboxylic acids, preferably poly(acrylic acid), poly(methacrylic acid) or poly(maleic acid); polymeric sulfonic acids, preferably poly(styrene sulfonic) acid or poly(vinylsulfonic) acid; or copolymers of vinycarboxylic acids and vinylsulfonic acids with other polymerizable monomers, preferably styrene and acrylic or methacrylic monomers.

7. Process for preparing an electrochemical sensor according to claims 1-6, **characterized in that** it comprises:
(A) obtaining, the first layer comprising a carbonaceous material forming the system of electrodes formed at least by a pseudo-reference electrode, a working electrode and a counter electrode and depositing said layer by printing, on the substrate;
(B) obtaining the second layer comprising a metal material and depositing said layer by printing only on the lower end of the pseudo-reference electrode;
(C) obtaining the third layer comprising insulating material and depositing said layer by printing on the part of the system of electrodes located between the analysis surface and the electrical contacts of the measuring equipment such that it leaves only the lower part of the electrodes of the system of electrodes exposed;and
(D) obtaining the fourth layer comprising polythiophene by means of a method selected from (D1), (D2) and (D3), wherein:
(D1) comprises obtaining the layer comprising a polythiophene and depositing said layer by printing on the lower end of the working electrode and then printing the layer comprising a non-conductive polymer gel on the layer comprising a polythiophene, wherein the polymer gel comprises acetylcholinesterase;
(D2) comprises obtaining the layer of conductive polymer gel comprising a non-conductive polymer gel and a polythiophene and depositing said layer by printing on the lower end of the working electrode, wherein the polymer gel comprises acetylcholinesterase; and
(D3) comprises obtaining the layer comprising a polythiophene and depositing said layer by printing on the lower end of the working electrode and then obtaining the layer comprising the magnetic nanoparticles, functionalized with acetylcholinesterase covalently bound on their surface, on the layer comprising a polythiophene,
wherein obtaining the fourth layer comprises: the application of either aqueous or solvent-based true solutions, colloidal dispersions or stable dispersions of finely divided particles of polythiophene previously obtained by means of oxidative polymerization.

8. Process according to claim 7, **characterized in that**, when the fourth layer is obtained by means of method D3, a magnet may be coupled below the substrate previously.

## Patentansprüche

1. Elektrochemischer Sensor für den Nachweis von Analyten in flüssigen Medien, welcher umfasst:
(a) eine erste Schicht (1), die aus einem kohlenstoffhaltigen Material besteht, die auf ein Substrat gedruckt ist, wobei die Schicht das Elektrodensystem des elektrochemischen Sensors bildet, welches sich wenigstens aus einer Pseudo-Referenzelektrode, einer Arbeitselektrode und einer Gegenelektrode zusammensetzt; wobei das kohlenstoffhaltige Material die Geometrie des Elektrodensystems abgrenzt;
(b) eine zweite Schicht (2), die ein Metallmaterial umfasst, die nur auf das untere Ende der Pseudo-Referenzelektrode gedruckt ist;
(c) eine dritte Schicht (3), die ein Isoliermaterial umfasst, die auf einen Teil des Elektrodensystems gedruckt ist, wobei der Teil derjenige ist, der sich zwischen der Analyseoberfläche (3a) und den elektrischen Kontakten (3b) der Messeinrichtung befindet, so dass nur der untere Teil der Elektroden des Elektrodensystems exponiert ist; und
(d) eine vierte Schicht (4), die Polythiophen umfasst, das zuvor mittels oxidativer Polymerisation erhalten wurde, welches gleichzeitig als Vermittler und Leiter wirkt, die nur auf das untere Ende der Arbeitselektrode gedruckt ist, ausgewählt aus (d1), (d2) und (d3), wobei:
d1) eine Schicht, die ein Polythiophen umfasst, die auf das untere Ende der Arbeitselektrode gedruckt ist, und eine Schicht, die ein nichtleitendes Polymergel umfasst, die auf der Polythiophenschicht abgeschieden ist, umfasst, wobei das Polymergel Acetylcholinesterase umfasst;
d2) eine Schicht aus leitendem Polymergel ist, die aus einem Polythiophen und einem nichtleitenden Polymergel besteht, wobei das Polymergel Acetylcholinesterase umfasst; und
d3) eine Schicht, die ein Polythiophen umfasst, die auf das untere Ende der Arbeitselektrode gedruckt ist, und eine Schicht, die magnetische Nanopartikel umfasst, die mit einer auf ihrer Oberfläche kovalent gebundenen Acetylcholinesterase funktionalisiert sind, die auf der Polythiophenschicht abgeschieden ist, umfasst.

2. Sensor gemäß Anspruch 1, **dadurch gekennzeichnet, dass**, wenn die vierte Schicht (4) die Schicht (d3) ist, sie zusätzlich einen unter dem Substrat angekoppelten Magneten umfassen kann.

3. Sensor gemäß einem der Ansprüche 1 und 2, **dadurch gekennzeichnet, dass** das Polythiophen sich wiederholende Struktureinheiten der Formel (I) umfasst, 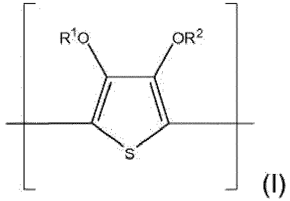 wobei R¹ und R² unabhängig eine C₁-C₁₂-Alkylgruppe sind oder eine C₁-C₁₂-1,n-Alkylengruppe bilden, wobei n = 1-12, gegebenenfalls substituiert durch ein C₁-C₁₂-Alkyl, C₂-C₁₂-Alken, Vinylen, Benzyl, Phenyl, eine Halogengruppe oder durch eine funktionelle Ester-, Amino-, Amido- oder Ethergruppe, die gegebenenfalls durch eine C₁-C₁₂-Alkylgruppe substituiert ist.

4. Sensor gemäß Anspruch 3, **dadurch gekennzeichnet, dass** in dem Polythiophen die R¹⁻ und R²⁻Gruppen eine Alkylengruppe, ausgewählt aus Methylen, 1,2-Ethylen und 1,3-Propylen, bilden.

5. Sensor gemäß Anspruch 3, **dadurch gekennzeichnet, dass** das Polythiophen einen anionischen Dotierstoff umfasst.

6. Sensor gemäß Anspruch 5, **dadurch gekennzeichnet, dass** der anionische Dotierstoff ein anorganisches Anion ausgewählt aus einem Sulfat-, Chlorid- und Bromidanion; ein organisches Anion mit Sulfonat- oder Phosphatgruppen, ausgewählt aus einer p-Toluolsulfonsäure und einer p-Toluolphosphonsäure; oder ein organisches Polyanion ausgewählt aus polymeren Carbonsäuren, vorzugsweise Poly(acrylsäure), Poly(methacrylsäure) oder Poly(maleinsäure); polymeren Sulfonsäuren, vorzugsweise Poly(styrolsulfon)säure oder Poly(vinylsulfon)säure; oder Copolymeren von Vinylcarbonsäuren und Vinylsulfonsäuren mit anderen polymerisierbaren Monomeren, vorzugsweise Styrol und Acryl- oder Methacrylmonomeren, ist.

7. Verfahren zum Herstellen eines elektrochemischen Sensors gemäß den Ansprüchen 1-6, **dadurch gekennzeichnet, dass** es umfasst:
(A) das Erhalten der ersten Schicht, die ein kohlenstoffhaltiges Material umfasst, die das Elektrodensystem bildet, das wenigstens durch eine Pseudo-Referenzelektrode, eine Arbeitselektrode und eine Gegenelektrode gebildet ist, und das Abscheiden der Schicht durch Drucken auf das Substrat;
(B) das Erhalten der zweiten Schicht, die ein Metallmaterial umfasst, und das Abscheiden der Schicht durch Drucken nur auf das untere Ende der Pseudo-Referenzelektrode;
(C) das Erhalten der dritten Schicht, die Isoliermaterial umfasst, und das Abscheiden der Schicht durch Drucken auf den Teil des Elektrodensystems, der sich zwischen der Analyseoberfläche und den elektrischen Kontakten der Messeinrichtung befindet, so dass sie nur den unteren Teil der Elektroden des Elektrodensystems exponiert lässt; und
(D) das Erhalten der vierten Schicht, die Polythiophen umfasst, mittels eines Verfahrens ausgewählt aus (D1), (D2) und (D3), wobei:
(D1) das Erhalten der Schicht, die ein Polythiophen umfasst, und das Abscheiden der Schicht durch Drucken auf das untere Ende der Arbeitselektrode und anschließend das Drucken der Schicht, die ein nichtleitendes Polymergel umfasst, auf die Schicht, die ein Polythiophen umfasst, umfasst, wobei das Polymergel Acetylcholinesterase umfasst;
(D2) das Erhalten der Schicht aus leitendem Polymergel, die ein nichtleitendes Polymergel und ein Polythiophen umfasst, und das Abscheiden der Schicht durch Drucken auf das untere Ende der Arbeitselektrode umfasst, wobei das Polymergel Acetylcholinesterase umfasst; und
(D3) das Erhalten der Schicht, die ein Polythiophen umfasst, und das Abscheiden der Schicht durch Drucken auf das untere Ende der Arbeitselektrode und anschließend das Erhalten der Schicht, die die magnetischen Nanopartikel umfasst, die mit auf ihrer Oberfläche kovalent gebundener Acetylcholinesterase funktionalisiert sind, auf der Schicht, die ein Polythiophen umfasst, umfasst,
wobei das Erhalten der vierten Schicht umfasst: das Aufbringen von entweder wässrigen oder lösungsmittelbasierten echten Lösungen, kolloidalen Dispersionen oder stabilen Dispersionen von fein verteilten Partikeln von Polythiophen, die zuvor mittels oxidativer Polymerisation erhalten wurden.

8. Verfahren gemäß Anspruch 7, **dadurch gekennzeichnet, dass**, wenn die vierte Schicht mittels Verfahren D3 erhalten wird, zuvor ein Magnet unter dem Substrat angekoppelt werden kann.

## Revendications

1. Capteur électrochimique pour la détection d'analytes dans un milieu liquide qui comprend :
(a) une première couche (1) constituée d'un matériau carboné imprimé sur un substrat, ladite couche formant le système d'électrodes du capteur électrochimique qui est composé d'au moins une électrode de pseudo-référence, une électrode de travail et une contre-électrode ; dans lequel le matériau carboné délimite la géométrie du système d'électrodes ;
(b) une deuxième couche (2) comprenant un matériau métallique imprimé uniquement sur l'extrémité inférieure de l'électrode de pseudo-référence ;
(c) une troisième couche (3) comprenant un matériau isolant imprimé sur une partie du système d'électrodes, ladite partie étant celle située entre la surface d'analyse (3a) et les contacts électriques (3b) de l'équipement de mesure, de sorte qu'uniquement la partie inférieure des électrodes du système d'électrodes est exposée ; et
(d) une quatrième couche (4) comprenant un polythiophène, préalablement obtenue au moyen d'une polymérisation oxydative, qui agit simultanément comme médiateur et conducteur, imprimée uniquement sur l'extrémité inférieure de l'électrode de travail sélectionnée parmi (d1), (d2) et (d3), dans lequel :
d1) comprend une couche comprenant un polythiophène imprimé sur l'extrémité inférieure de l'électrode de travail et une couche comprenant un gel polymère non conducteur déposé sur ladite couche de polythiophène, dans lequel le gel polymère comprend une acétylcholinestérase ;
d2) est une couche de gel polymère conducteur constituée d'un polythiophène et d'un gel polymère non conducteur, dans lequel le gel polymère comprend une acétylcholinestérase ; et
d3) comprend une couche comprenant un polythiophène imprimé sur l'extrémité inférieure de l'électrode de travail et une couche comprenant des nanoparticules magnétiques, fonctionnalisées avec une acétylcholinestérase liée par liaison covalente sur leur surface, déposées sur ladite couche de polythiophène.

2. Capteur selon la revendication 1, **caractérisé en ce que**, quand la quatrième couche (4) est la couche (d3), elle peut comprendre en outre un aimant couplé en dessous du substrat.

3. Capteur selon l'une quelconque des revendications 1 et 2, **caractérisé en ce que** le polythiophène contient des motifs structurels répétitifs de formule (I), 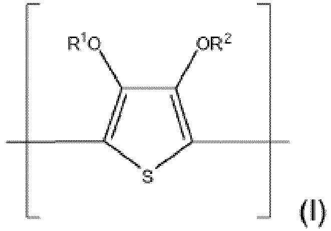 dans laquelle R¹ et R² sont indépendamment un groupe alkyle en C₁ à C₁₂ ou forment un groupe 1,n-alkylène en C₁ à C₁₂, avec n = 1 à 12, facultativement substitué avec un groupe alkyle en C₁ à C₁₂, alcène en C₂ à C₁₂, vinylène, benzyle, phényle, halogène, ou avec un groupe fonctionnel ester, amino, amido ou éther facultativement substitué avec un groupe alkyle en C₁ à C₁₂.

4. Capteur selon la revendication 3, **caractérisé en ce que** dans le polythiophène les groupes R¹ et R² forment un groupe alkylène sélectionné parmi le méthylène, le 1,2-éthylène et le 1,3-propylène.

5. Capteur selon la revendication 3, **caractérisé en ce que** le polythiophène comprend un dopant anionique.

6. Capteur selon la revendication 5, **caractérisé en ce que** le dopant anionique est un anion inorganique sélectionné parmi un anion sulfate, chlorure et bromure ; un anion organique ayant des groupes sulfonate et phosphate sélectionné parmi un acide p-toluènesulfonique et un acide p-toluènephosphonique ; ou un polyanion organique sélectionné parmi les acides carboxyliques polymères, de préférence un poly(acide acrylique), un poly(acide méthacrylique) ou un poly(acide maléique) ; les acides sulfoniques polymères, de préférence un polyacide (styrène sulfonique) ou un polyacide (vinylsulfonique) ; ou les copolymères d'acides vinycarboxyliques et d'acides vinylsulfonique avec d'autres monomères polymérisables, de préférence des monomères de styrène et acryliques ou méthacryliques.

7. Procédé de préparation d'un capteur électrochimique selon les revendications 1 à 6, **caractérisé en ce qu'**il comprend :
(A) l'obtention de la première couche comprenant un matériau carboné formant le système d'électrodes formé au moins d'une électrode de pseudo-référence, d'une électrode de travail et d'une contre-électrode et le dépôt de ladite couche par impression, sur le substrat ;
(B) l'obtention de la deuxième couche comprenant un matériau métallique et le dépôt de ladite couche par impression uniquement sur l'extrémité inférieure de l'électrode de pseudo-référence ;
(C) l'obtention de la troisième couche comprenant un matériau isolant et le dépôt de ladite couche par impression sur la partie du système d'électrodes située entre la surface d'analyse et les contacts électriques de l'équipement de mesure de façon qu'elle laisse uniquement la partie inférieure des électrodes du système d'électrodes exposée ; et
(D) l'obtention de la quatrième couche comprenant un polythiophène au moyen d'un procédé sélectionné parmi (D1), (D2) et (D3), dans lequel :
(D1) comprend l'obtention de la couche comprenant un polythiophène et le dépôt de ladite couche par impression sur l'extrémité inférieure de l'électrode de travail et ensuite l'impression de la couche comprenant un gel polymère non conducteur sur la couche comprenant un polythiophène, dans lequel le gel polymère comprend une acétylcholinestérase ;
(D2) comprend l'obtention de la couche de gel polymère conducteur comprenant un gel polymère non conducteur et un polythiophène et le dépôt de ladite couche par impression sur l'extrémité inférieure de l'électrode de travail, dans lequel le gel polymère comprend une acétylcholinestérase ; et
(D3) comprend l'obtention de la couche comprenant un polythiophène et le dépôt de ladite couche par impression sur l'extrémité inférieure de l'électrode de travail et ensuite l'obtention de la couche comprenant les nanoparticules magnétiques, fonctionnalisées avec une acétylcholinestérase liée par liaison covalente sur leur surface, sur la couche comprenant un polythiophène,
dans lequel l'obtention de la quatrième couche comprend : l'application de solutions authentiques, de dispersions colloïdales ou de dispersions stables, à base d'eau ou d'un solvant, de particules finement divisées de polythiophène préalablement obtenues au moyen d'une polymérisation oxydative.

8. Procédé selon la revendication 7, **caractérisé en ce que**, quand la quatrième couche est obtenue au moyen du procédé D3, un aimant peut être préalablement couplé en dessous du substrat.
